# EUROPEAN PATENT APPLICATION

(11) **EP 2 832 392 A1**
(43) Date of publication of application: **04.02.2015**
(21) Application number: 13178343.3
(22) Date of filing: 29.07.2013
(51) Int. Cl.: A61M 11/00, A61M 15/00, A61M 15/08

(54) **Aerosol delivery device and method for operating an aerosol delivery device**

(71) Applicant: PARI Pharma GmbH, 82319 Starnberg (DE)
(72) Inventor: Roeder, Sascha, 80636 München (DE); Schusching, Uwe, 81371 München (DE)
(74) Representative: Hoffmann Eitle

(57) **Abstract**

The invention relates to an aerosol delivery device (10, 100, 100') comprising an aerosol generator (12, 106) for generating an aerosol in the device and a fluid conveying unit (14, 16, 18; 102, 104, 130) configured to induce a first fluid flow (26, 120) having a first flow rate in the device for transporting at least a portion of the generated aerosol outside the device and to induce a second fluid flow (32, 122) having a second flow rate in the device for conveying a fluid into the device, wherein the fluid conveying unit comprises a vibrator (18, 104) for vibrating the aerosol and the first flow rate is equal to or smaller than the second flow rate. Further, the invention relates to a method for operating an aerosol delivery device (10, 100, 100'), comprising the steps of generating a predetermined amount of an aerosol in the device, inducing a first fluid flow (26, 120) having a first flow rate in the device for transporting at least a portion of the generated aerosol outside the device, inducing a second fluid flow (32, 122) having a second flow rate in the device for conveying a fluid into the device and vibrating the aerosol, wherein the first flow rate is equal to or smaller than the second flow rate.

## Description

### Field of the Invention

The invention relates to an aerosol delivery device (nebuliser) and a method for operating this aerosol delivery device.

### Background Art

Diseases and conditions affecting either paranasal sinuses or both the nasal cavity and the paranasal sinuses, in particular acute and chronic forms of rhinosinusitis, are increasing in incidence and prevalence in many countries and regions of the world, including Europe and the United States. These conditions may be associated with significant symptoms and have a negative impact on quality of life and daily functioning.

The method most commonly used to deliver medications to the nasal cavity is a squeeze bottle or a metering spray pump nebulising volumes of 50 to 140 µl per actuation. However, studies investigating the *in vivo* deposition pattern of droplets administered by a spray pump indicate that local distribution is primarily in the anterior portion of the nasal cavity leaving large portions of the nasal cavity unexposed to drug (see Suman et al., "Comparison of nasal deposition and clearance of aerosol generated by a nebuliser and an aqueous spray pump", Pharmaceutical Research, Vol. 16, No. 10, 1999). Furthermore, drugs applied by nasal pump sprays are cleared very fast from the nose, an average clearance time of between 10 and 20 minutes being accepted as normal (see C. Marriott, "Once-a-Day Nasal Delivery of Steroids: Can the Nose Be Tricked?" RDD Europe 2007, proceedings p.179-185).

The fast clearance rate of the nose and the difficulties to overcome these disadvantages by an increase of the solution viscosity have also been described by Pennington et al. ("The influence of solution viscosity on nasal spray deposition and clearance", Intern. Journal of Pharmaceutics, 43, p. 221-224, 1988). However, those attempts were only successful to improve retention of drugs in the nose prolonging the residence time, the time to clear 50% of dose, up to 2.2 hours. Consequently, the effective treatment of the nasal and paranasal mucosa via a method to increase residence time remains challenging.

While the mucosa of the nasal cavity is a feasible target for locally administered drugs formulated as nasal sprays, the sinuses and the osteomeatal complex are not easily accessed by liquid formulations. In the case of relatively coarse aerosols, such as conventional nasal sprays, the deposition on the sinus mucosa is negligible, and even finer aerosols, such as those generated by nebulisers, exhibit a very low degree of sinus deposition.

The primary reason for the lack of access of an inhaled aerosol to the sinuses is anatomical: in contrast to the nasal cavity, the sinuses are not actively ventilated. The latter are connected to the nasal passage via small orifices called ostia, whose diameter is typically in the region of about 0.5 to 3.0 mm for a healthy person and up to about 10 mm for a patient after sinus surgery (functional endoscopic sinus surgery). When air is inhaled through the nose and passes through the nasal passage into the trachea, there is only very little convective flow into the ostia.

To address the need for devices and methods which are more effective in delivering an aerosol to the osteomeatal complex and paranasal sinuses, it was suggested in WO 2005/023335 that certain particle size and vorticity characteristics must be achieved in order that a majority of an aerosolised drug formulation reaches the deep nasal cavities and the sinuses. Furthermore, WO 2004/020029 discloses an aerosol generator comprising a nebuliser and a compressor which delivers a pulsating stream of air to the nebuliser. In use of this aerosol generator, the main aerosol flow supplied to a patient's nostril is superimposed by pressure fluctuations in order to improve the aerosol deposition efficiency in the paranasal sinuses. This document further describes that the aerosol emitted from the nebuliser should be introduced through one nostril via an appropriate nosepiece with closed soft palate, and that the contralateral nostril must be occluded by an appropriate flow resistance device.

A substantial further improvement was achieved through the teaching of EP 1 820 493 A2 according to which the sinunasal deposition of a pulsating aerosol can be significantly increased if it is ensured that the pressure fluctuation maintains a certain amplitude, such as at least about 5 mbar pressure difference. The used frequencies are around 10 Hz to 90 Hz.

US-A-2008/0251068 discloses an aerosol therapy device comprising a nebuliser for generating an aerosol and forming an aerosol flow, a first nosepiece for introducing the aerosol flow into one of the two nostrils of a user, a pressure fluctuation source for generating a pressure fluctuation, a second nosepiece for introducing the pressure fluctuation into the other of the two nostrils of the user in order to superimpose the pressure fluctuation and the aerosol flow, and a sensor system comprising a first pressure sensor for detecting the signal of the pressure fluctuation that arrives at the first nosepiece, and an evaluation means that concludes the degree of closure of the velum (soft palate) of the user based on the signal. In this way, it can be ensured that aerosol application in the nose only occurs if the velum of the user is closed, so that an erroneous deposition of the aerosol in the pharyngeal cavity and the lungs of the user can be prevented.

FR-A-2 938 770 discloses an aerosol delivery device comprising an aerosol generator, a mechanical means for generating a constant gas flow and a vibrator for vibrating the gas flow. A certain flow of aerosol generated by the aerosol generator is transported into one nostril of a user and vibrated by the vibrator. In order to establish a closed gas circuit, the device is also connected to the other nostril of the user and a predetermined flow of gas which is equal to the flow entering the one nostril is extracted from the other nostril with the mechanical means.

However, the gas flow supplied to the one nostril is a superposition of the constant flow generated by the mechanical means and the time variable flow generated by the vibrator, while the gas flow extracted from the other nostril is constituted only by the constant flow generated by the mechanical means. Thus, the gas flow supplied to the one nostril is periodically larger than the gas flow extracted from the other nostril, leading to the undesired deposition of a fraction of the transported aerosol in the pharyngeal cavity and the lungs of the user.

Therefore, there remains a need for a simplified aerosol delivery device and method, preventing the erroneous deposition of aerosols in the pharyngeal cavity and the lungs of a user, while eliminating the requirement of a closure of the velum (soft palate) during aerosol delivery.

### Summary of the Invention

One object of the invention is to provide a simplified aerosol delivery device which allows for the erroneous deposition of aerosols in the pharyngeal cavity and the lungs of a user to be prevented, while eliminating the requirement of a closure of the soft palate. Further, the invention aims to provide a method for operating this aerosol delivery device.

These goals are achieved by an aerosol delivery device with the technical features of claim 1 and a method for operating an aerosol delivery device with the technical features of claim 9. Preferred embodiments of the invention follow from the dependent claims.

The invention provides an aerosol delivery device comprising an aerosol generator for generating an aerosol in the device, and a fluid conveying unit configured to induce a first fluid flow having a first flow rate in the device for transporting at least a portion of the generated aerosol outside the device and to induce a second fluid flow having a second flow rate in the device for conveying a fluid into the device, wherein the fluid conveying unit comprises a vibrator for vibrating the aerosol, and the first flow rate is equal to or smaller than the second flow rate.

As used herein, the term "vibration (pulsation, pressure oscillation, pressure fluctuation) of an aerosol" is understood as a periodic change of pressure that occurs at a predetermined frequency. Preferably, the vibration is regular, i.e., the time interval between pressure peaks is approximately constant. By vibrating the aerosol at a given frequency, aerosol diffusion can be significantly enhanced, thus enabling improved access to locations that are difficult to reach with a constant pressure aerosol flow, such as the paranasal sinuses. Additionally, pressure differences between nasal and sinus cavity effectuate an air flow and, with it, ventilation of the sinuses. The principle of vibrating an aerosol for enhanced sinus deposition has recently been found and is described, for example, in WO 2004/020029.

Since in the aerosol delivery (generation, inhalation) device according to the present invention, the fluid conveying unit is configured to induce the first fluid flow having the first flow rate in the device for transporting at least a portion of the generated aerosol outside the device and to induce the second fluid flow having the second flow rate in the device for conveying a fluid into the device, wherein the first flow rate is equal to or smaller than the second flow rate, it can be ensured that the first flow rate is permanently equal to or smaller than the second flow rate, i.e., throughout an operation cycle or an aerosol deposition cycle of the aerosol delivery device. Hence, it can be guaranteed that within any time interval the incremental amount of fluid transported outside the device, e.g., into a patient's first nostril, is always equal to or smaller than the amount of fluid conveyed from outside the device into the device, e.g., from the patient's second nostril, so that the deposition of an aerosol in the pharyngeal cavity and the lungs of the patient can be reliably and efficiently prevented.

In particular, the first fluid flow may be supplied to the first nostril of a patient through a first nosepiece or the like and the second fluid flow may be extracted from the second nostril of the patient through a second nosepiece or the like. In this way, a closed fluid circuit between the device and the patient may be established.

The aerosol transported in the first fluid flow can be vibrated by means of the vibrator, thereby enhancing aerosol deposition in locations which are difficult to reach, such as the paranasal sinuses, as has been detailed above.

The first fluid flow may be a gas flow, such as an air flow. In the case of an air flow, the first fluid flow can be provided in a particularly simple manner with a gas conveying element, such as a pump or the like, using, for example, ambient air, so that no separate gas reservoir is required. Further, the second fluid flow may be a gas flow, such as an air flow, which may contain aerosol particles from the nose.

The second flow rate may be 60 l/min or less, 30 l/min or less, 20 l/min or less, 10 l/min or less, 5 l/min or less or 3 l/min or less. The first flow rate may be 60 l/min or less, 30 l/min or less, 20 l/min or less, 10 l/min or less, 5 1/min or less or 3 1/min or less, as long as the first flow rate is equal to or smaller than the second flow rate. The second fluid flow may be a constant fluid flow.

The aerosol generator may be a nebuliser, such as an ultrasonic nebuliser, a vibrating membrane nebuliser, e.g., an electronic vibrating membrane nebuliser, a jet nebuliser or the like. For the use of a vibrating membrane nebuliser, it is to be understood that devices of this type only generate the aerosol and have no influence on the vibration imparted to the transported aerosol by the vibrator. These two kinds of vibration types are separate from each other and may differ in their parameters, such as amplitude, frequencies, wave forms and oscillations.

If the aerosol generator is a vibrating membrane nebuliser, no transport flow is required for the generation of an aerosol so that the aerosol generation and the first fluid flow are entirely independent from each other. Therefore, the first fluid flow can be precisely controlled, thus further improving the aerosol deposition efficiency.

The vibrator may be configured so that the duration of vibrating the aerosol may be equal to or less than 15.0 s and preferably lie in the range of 0.1 to 15.0 s, more preferably in the range of 0.1 to 10.0 s, even more preferably in the range of 0.1 to 1.0 s and yet more preferably in the range of 0.5 to 1.0 s.

A target area to be therapeutically treated may be the nasal cavity, the mucosa in the nose, the osteomeatal complex or a paranasal sinus. The paranasal sinuses consist of four pairs of air-filled cavities or spaces within the bones of the skull and face. They are divided into subgroups which are named according to the bones they lie under: (1) the maxillary sinuses, also called the antra, which are located under the eyes, in the upper jawbone; (2) the frontal sinuses, which lie above the eyes, in the bone of the forehead; (3) the ethmoid sinuses, positioned between the nose and the eyes, backwards into the skull; and (4) the sphenoid sinuses, which are more or less in the centre of the skull base. While the primary function of the sinuses is not entirely clear, it appears that they decrease the relative weight of the front of the skull, warm and humidify the inhaled air before it reaches the lungs, increase the resonance of the voice, and perhaps provide a buffer against blows to the face.

The nasal cavity and the paranasal sinuses are lined with mucosa. Mucosae, or mucous membranes, are mucus-covered epithelial linings. The mucosae of the nasal cavity and the paranasal sinuses are often affected by conditions such as allergies and infections, and the aerosol delivery device of the present invention provides improved means to deliver aerosols comprising therapeutically useful active agents to these membranes.

As mentioned above and described in detail in WO 2004/020029, a vibrating aerosol enters the paranasal sinuses after nasal inhalation to a much larger extent than a conventional aerosol having a substantially constant pressure, provided that appropriate particle (i.e., aerosol droplet) sizes are selected. Larger particle sizes will lead to little sinus deposition, but to a large deposition on the nasal mucosa, whereas very small particle sizes allow the aerosol droplets to enter the sinuses following the pressure gradient of a pressure pulse, but also to exit from the sinuses again without being deposited therein.

The paranasal sinuses are, under normal circumstances, poorly ventilated during breathing. Most of the air exchange of the sinuses occurs by diffusion of air via the ostia, whereas little or no convective flow is observed. If an aerosol, such as a therapeutic aerosol generated by a conventional nebuliser, is inhaled through the nose, the aerosol will flow through the nasal cavity to the lower respiratory tract if it comprises particles with a sufficiently small diameter. Since there is virtually no active flow into the paranasal sinuses, very little or almost none of the aerosol is deposited therein.

In contrast, an aerosol which vibrates creates periodic transient pressure gradients extending from the actively ventilated nasal cavity through the ostia to the sinuses, which gradients cause a short period of convective flow of air and aerosol into the sinuses until the pressure therein has become equal to the air pressure in the nasal cavity. A portion of the aerosol droplets which thus enter the paranasal sinuses is deposited therein onto the mucosa. The extent to which the aerosol is deposited depends e.g. on the droplet size. For example, very small droplets, such as droplets below 1 µm in diameter, are likely to be deposited with lower concentrations in the sinuses during the residence time and be partly expelled from the sinuses during the subsequent vibration phase, in which the aerosol pressure, and thus the pressure in the nasal cavity, is lower than the pressure within the sinuses, and during which a convective flow of air from the sinuses to the nasal cavity occurs. Preferably, the aerosol generator of the aerosol delivery device according to the present invention is configured to generate an aerosol with a particle size (diameter) in a range of 1 to 10 µm, more preferably in the range of 1 to 5 µm.

The used aerosol may have a high potential to bring a sufficient amount of aerosol to a desired location outside the aerosol delivery device, such as the paranasal sinuses. The present invention works similar with smaller aerosol particles under 1 µm.

The aerosol may be a pharmaceutical aerosol for the delivery of an active compound. An active compound is a natural, biotechnology-derived or synthetic compound or mixture of compounds useful for the diagnosis, prevention, management, or treatment of a disease, condition, or symptom of an animal, in particular a human. Other terms which may be used as synonyms of active compound include, for example, active ingredient, active pharmaceutical ingredient, drug substance, drug, and the like.

The active compound comprised in the aerosol used for the device and the method of the invention may include a drug substance which is useful for the prevention, management, or treatment of any disease, symptom, or condition affecting the nose, the sinuses and/or the osteomeatal complex.

Among the active compounds which may be useful for serving one of these purposes are, for example, substances selected from the group consisting of anti-inflammatory compounds, glucocorticoids, anti-allergic drugs, antioxidants, vitamins, anti-infective agents, antibiotics, antifungals, antivirals, mucolytics, decongestants, antiseptics, immunomodulators, vaccines, wound healing agents, local anesthetics, oligonucleotides, peptides, proteins and plant extracts. Such compounds may be used in the form of a suspension, a solution, a colloidal formulation (i.e. liposomal), etc.

Examples of potentially useful anti-inflammatory compounds are glucocorticoids and non-steroidal anti-inflammatory agents such as betamethasone, beclomethasone, budesonide, ciclesonide, dexamethasone, desoxymethasone, fluocinolone acetonide, fluocinonide, flunisolide, fluticasone, icomethasone, rofleponide, triamcinolone acetonide, fluocortin butyl, hydrocortisone, hydroxycortisone-17-butyrate, prednicarbate, 6-methylprednisolone aceponate, mometasone furoate, dehydroepiandrosterone-sulfate (DHEAS), elastane, prostaglandin, leukotriene, bradykinin antagonists, non-steroidal anti-inflammatory drugs (NSAIDs), such as ibuprofen including any pharmaceutically acceptable salts, esters, isomers, stereoisomers, diastereomers, epimers, solvates or other hydrates, prodrugs, derivatives, or any other chemical or physical forms of active compounds comprising the respective active moieties.

Examples of anti-infective agents, whose class or therapeutic category is herein understood as comprising compounds which are effective against bacterial, fungal, and viral infections, i.e. encompassing the classes of antimicrobials, antibiotics, antifungals, antiseptics, and antivirals, are
- penicillins, including benzylpenicillins (penicillin-G-sodium, clemizone penicillin, benzathine penicillin G), phenoxypenicillins (penicillin V, propicillin), aminobenzylpenicillins (ampicillin, amoxycillin, bacampicillin), acylaminopenicillins (azlocillin, mezlocillin, piperacillin, apalcillin), carboxypenicillins (carbenicillin, ticarcillin, temocillin), isoxazolyl penicillins (oxacillin, cloxacillin, dicloxacillin, flucloxacillin), and amidine penicillins (mecillinam);
- cephalosporins, including cefazolins (cefazolin, cefazedone); cefuroximes (cefuroxim, cefamandole, cefotiam), cefoxitins (cefoxitin, cefotetan, latamoxef, flomoxef), cefotaximes (cefotaxime, ceftriaxone, ceftizoxime, cefmenoxime), ceftazidimes (ceftazidime, cefpirome, cefepime), cefalexins (cefalexin, cefaclor, cefadroxil, cefradine, loracarbef, cefprozil), and cefiximes (cefixime, cefpodoxim proxetile, cefuroxime axetil, cefetamet pivoxil, cefotiam hexetil), loracarbef, cefepim, clavulanic acid / amoxicillin, Ceftobiprole;
- synergists, including beta-lactamase inhibitors, such as clavulanic acid, sulbactam, and tazobactam;
- carbapenems, including imipenem, cilastin, meropenem, doripenem, tebipenem, ertapenem, ritipenam, and biapenem;
- monobactams, including aztreonam;
- aminoglycosides, such as apramycin, gentamicin, amikacin, isepamicin, arbekacin, tobramycin, netilmicin, spectinomycin, streptomycin, capreomycin, neomycin, paromoycin, and kanamycin;
- macrolides, including erythromycin, clarythromycin, roxithromycin, azithromycin, dithromycin, josamycin, spiramycin and telithromycin;
- gyrase inhibitors or fluroquinolones, including ciprofloxacin, gatifloxacin, norfloxacin, ofloxacin, levofloxacin, perfloxacin, lomefloxacin, fleroxacin, garenoxacin, clinafloxacin, sitafloxacin, prulifloxacin, olamufloxacin, caderofloxacin, gemifloxacin, balofloxacin, trovafloxacin, and moxifloxacin;
- tetracyclins, including tetracyclin, oxytetracyclin, rolitetracyclin, minocyclin, doxycycline, tigecycline and aminocycline;
- glycopeptides, inlcuding vancomycin, teicoplanin, ristocetin, avoparcin, oritavancin, ramoplanin, and peptide 4;
- polypeptides, including plectasin, dalbavancin, daptomycin, oritavancin, ramoplanin, dalbavancin, telavancin, bacitracin, tyrothricin, neomycin, kanamycin, mupirocin, paromomycin, polymyxin B and colistin;
- sulfonamides, including sulfadiazine, sulfamethoxazole, sulfalene, co-trimoxazole, co-trimetrol, co-trimoxazine, and co-tetraxazine;
- azoles, including clotrimazole, oxiconazole, miconazole, ketoconazole, itraconazole, fluconazole, metronidazole, tinidazole, bifonazol, ravuconazol, posaconazol, voriconazole, and ornidazole and other antifungals including flucytosin, griseofulvin, tolnaftat, naftifin, terbinafin, amorolfin, ciclopiroxolamin, echinocandins, such as micafungin, caspofungin, anidulafungin;
- nitrofurans, including nitrofurantoin and nitrofuranzone;
- polyenes, including amphotericin B, natamycin, nystatin, flucocytosine; flucytosine
- other antibiotics, including tithromycin, lincomycin, clindamycin, oxazolidinones (linzezolids), ranbezolid, streptogramine A+B, pristinamycin A+B, Virginiamycin A+B, dalfopristin /quinupristin (Synercid), chloramphenicol, ethambutol, pyrazinamid, terizidon, dapson, prothionamid, fosfomycin, fucidinic acid, rifampicin, isoniazid, cycloserine, terizidone, ansamycin, lysostaphin, iclaprim, mirocin B17, clerocidin, filgrastim, and pentamidine;
- antivirals, including aciclovir, ganciclovir, birivudin, valaciclovir, zidovudine, didanosin, thiacytidin, stavudin, lamivudin, zalcitabin, ribavirin, nevirapirin, delaviridin, trifluridin, ritonavir, saquinavir, indinavir, foscarnet, amantadin, podophyllotoxin, vidarabine, tromantadine, and proteinase inhibitors;, si RNA based drugs
- antiseptics, including acridine derivatives, iodine-povidone, benzoates, rivanol, chlorhexidine, quarternary ammonium compounds, cetrimides, biphenylol, clorofene, taurolidine and octenidine;
- plant extracts or ingredients, such as plant extracts from chamomile, hamamelis, echinacea, calendula, thymian, bromelain, papain, pelargonium, pine trees, essential oils, myrtol, pinen, limonen, cineole, thymol, mentol, camphor, tannin, alpha-hederin, bisabolol, lycopodin, vitapherole;
- wound healing compounds including dexpantenol, allantoin, vitamins, hyaluronic acid, alpha-antitrypsin, anorganic and organic zinc salts/compounds, salts of bismuth and selen
- interferones (alpha, beta, gamma), tumor necrosis factors, cytokines, interleukines;
- immunmodulators including methotrexat, azathioprine, cyclosporine, tacrolimus, sirolimus, rapamycin, mofetil; mofetil-mycophenolate.
- cytostatics and metastasis inhibitors;
- alkylants, such as nimustine, melphanlane, carmustine, lomustine, cyclophosphosphamide, ifosfamide, trofosfamide, chlorambucil, busulfane, treosulfane, prednimustine, thiotepa;
- antimetabolites, e.g. cytarabine, fluorouracil, methotrexate, mercaptopurine, tioguanine;
- alkaloids, such as vinblastine, vincristine, vindesine;
- antibiotics, such as alcarubicine, bleomycine, dactinomycine, daunorubicine, doxorubicine, epirubicine, idarubicine, mitomycine, plicamycine;
- complexes of transition group elements (e.g. Ti, Zr, V, Nb, Ta, Mo, W, Pt) such as carboplatinum, cis-platinum and metallocene compounds such as titanocendichloride;
- amsacrine, dacarbazine, estramustine, etoposide, beraprost, hydroxycarbamide, mitoxanthrone, procarbazine, temiposide;
- paclitaxel, iressa, zactima, poly-ADP-ribose-polymerase (PRAP) enzyme inhibitors, banoxantrone, gemcitabine, pemetrexed, bevacizumab, ranibizumab.

Examples of potentially useful mucolytics are DNase (including dornase alpha), P2Y2-agonists (denufosol), drugs affecting chloride and sodium permeation, such as N-(3,5-Diamino-6-chloropyrazine-2-carbony)-N'-{4-[4-(2,3-dihydroxypropoxy)-phenyl]butyl}guanidine methanesulfonate (PARION 552-02), heparinoids, guaifenesin, acetylcysteine, carbocysteine, ambroxol, bromhexine, tyloxapol, lecithins, myrtol, and recombinant surfactant proteins.

Examples of potentially useful vasoconstrictors and decongestants which may be useful to reduce the swelling of the mucosa are phenylephrine, naphazoline, tramazoline, tetryzoline, oxymetazoline, fenoxazoline, xylometazoline, epinephrine, isoprenaline, hexoprenaline, and ephedrine. Examples of potentially useful local anaesthetic agents include benzocaine, tetracaine, procaine, lidocaine and bupivacaine.

Examples of potentially useful antiallergic agents include the afore-mentioned glucocorticoids, cromolyn sodium, nedocromil, cetrizin, loratidin, roflumilast, ziluton, omalizumab, heparinoids and other antihistamins, including azelastine, cetirizin, desloratadin, ebastin, fexofenadin, levocetirizin, loratadin.

Antisense oligonucleotides are short synthetic strands of DNA (or analogs) that are complimentary or antisense to a target sequence (DNA, RNA) designed to halt a biological event, such as transcription, translation or splicing. The resulting inhibition of gene expression makes oligonucleotides dependent on their composition useful for the treatment of many diseases and various compounds are currently clinically evaluated.

Also si RNA can be applied as an active compound.

Examples of potentially useful peptides and proteins include antibodies against toxins produced by microorganisms, antimicrobial peptides such as cecropins, defensins, thionins, and cathelicidins.

Also immunoglobulins (e.g., IgG, IgE, IgD, IgA, IgM, IgY, IgW) or fragments of immunoglobulins (like IgG fragments, e.g., Fab, Fc, F[ab]2, Fc, Facb, pFc) as well as polyclonal antibodies, monoclonal antibodies and recombinant antibodies are potentially applicable antibodies.

The fluid conveying unit may comprise one or more fluid conveying elements, such as pumps, compressors, compressed air supplies, turbines and/or ventilators (with or without included work modes like BiPAP, CPAP, ASB, PAV and so on), which work with or without Venturi principle, gas valve(s), gas controller, regulator, actuator, interrupter, switch three way selector valve or the like.

In one embodiment, the fluid conveying unit further comprises a first fluid conveying element, such as a pump, configured to induce a third fluid flow having a third flow rate in the device and a second fluid conveying element, such as a pump, configured to induce the second fluid flow in the device, wherein the vibrator is configured to vibrate the third fluid flow so as to form or induce the first fluid flow in the device. Hence, the first fluid flow is formed by superimposing pressure fluctuations onto the third fluid flow by the vibrator.

The third flow rate is smaller than the second flow rate. The pressure fluctuations, i.e., the time variable flow superimposed by the vibrator onto the third fluid flow, are compensated by the smaller third flow rate of the third fluid flow as compared to the second flow rate of the second fluid flow, thus reliably preventing any aerosol deposition in the pharyngeal cavity and the lungs of a patient.

The third flow rate may be 55 l/min or less, 30 l/min or less, 20 l/min or less, 10 l/min or less, 5 l/min or less or 3 l/min or less, as long as the third flow rate is smaller than the second flow rate. The second fluid flow and/or the third fluid flow may be constant fluid flows.

The same type or different types of fluid conveying elements may be used for the first and second fluid conveying elements, as long as these elements are configured so that the first flow rate is equal to or smaller than the second flow rate.

In this embodiment, the second fluid flow and the third fluid flow are provided by two separate fluid conveying elements, so that the second flow rate and the third flow rate can be adjusted independently of each other. Hence, the second and third flow rates and, thus, also the first flow rate can be adjusted in a particularly simple and reliable manner. For example, each of the first and second fluid conveying elements may be constituted by a pump, wherein the pump constituting the first fluid conveying element has a smaller flow rate or pumping capacity than the pump constituting the second fluid conveying element.

The vibrator may be configured to vibrate the third fluid flow with a predetermined vibration amplitude and the vibration amplitude may be equal to or smaller than the difference between the second and third flow rates. The vibration amplitude may be in a range from 1 to 40 l/min, 3 to 30 l/min, 5 to 25 l/min or 10 to 20 l/min.

In this way, in particular if the second and third fluid flows are constant fluid flows, any deposition of aerosol in the pharyngeal cavity and the lungs of a patient is prevented in a particularly reliable, efficient and simple manner.

Alternatively, the second fluid conveying element may be configured to induce a fourth fluid flow having a fourth flow rate in the device and the vibrator may be configured to vibrate the fourth fluid flow so as to form or induce the second fluid flow in the device. In this case, the vibrations imparted by the vibrator to the third fluid flow and the fourth fluid flow may be phase shifted by 180°.

Hence, by choosing a third flow rate which is equal to or smaller than the fourth flow rate, it can be ensured that the first flow rate is equal to or smaller than the second flow rate throughout an operation cycle of the aerosol delivery device, thereby enabling a reliable prevention of erroneous aerosol deposition in the pharyngeal cavity and the lungs of a patient. In such a device configuration, a twin head pump as will be described in detail below may be particularly advantageously used as the vibrator.

In another embodiment, the fluid conveying unit comprises, further to the vibrator, a fluid conveying element, such as a pump, configured to induce a third fluid flow having a third flow rate and a fourth fluid flow having a fourth flow rate in the device, wherein the vibrator is configured to vibrate the third fluid flow so as to form or induce the first fluid flow in the device and to vibrate the fourth fluid flow so as to form or induce the second fluid flow in the device. The fluid conveying element may be a single fluid conveying element.

Hence, the first fluid flow is formed by superimposing pressure fluctuations onto the third fluid flow by the vibrator and the second fluid flow is formed by superimposing pressure fluctuations onto the fourth fluid flow by the vibrator.

The third flow rate may be equal to the fourth flow rate, e.g., if a pump is used as the fluid conveying element, the third fluid flow is induced by an outlet of the pump and the fourth fluid flow is induced by an inlet of the pump.

Further, the vibrator may be configured so that the vibration of the third fluid flow and the vibration of the fourth fluid flow are phase shifted by 180°. In particular, the vibrator may have an outlet which is coupled to the third fluid flow and an inlet which is coupled to the fourth fluid flow. For example, a twin head pump as will be described in detail below may be used as the vibrator, wherein the pumping operation of a first piston acts on the third fluid flow and the pumping operation of a second piston acts on the fourth fluid flow.

If the third flow rate is equal to or smaller than the fourth flow rate and the vibration of the third fluid flow and the vibration of the fourth fluid flow are phase shifted by 180°, it can be ensured in a simple and reliable manner that the first flow rate will be equal to or smaller than the second flow rate throughout an operation cycle or an aerosol deposition cycle of the aerosol delivery device. Hence, an aerosol deposition in the pharyngeal cavity and the lungs of a patient can be reliably prevented, even if the vibration amplitude is significantly larger than the difference between the third and fourth flow rates. Therefore, the aerosol delivery device according to this embodiment can be particularly advantageously used for applications in which large vibration amplitudes are required.

In another embodiment, the fluid conveying unit comprises, further to the vibrator, a fluid conveying element, such as a pump, configured to induce a third fluid flow having a third flow rate and a fourth fluid flow having a fourth flow rate in the device and a flow conversion element configured to convert the third fluid flow into a fifth fluid flow having a fifth flow rate, wherein the vibrator is configured to vibrate the fifth fluid flow so as to form or induce the first fluid flow in the device. Hence, the first fluid flow is formed by superimposing pressure fluctuations onto the fifth fluid flow by the vibrator. The fluid conveying element may be a single fluid conveying element.

The third flow rate may be equal to the fourth flow rate, e.g., if a pump is used as the fluid conveying element, the third fluid flow is induced by an outlet of the pump and the fourth fluid flow is induced by an inlet of the pump. The fifth flow rate may be smaller than the third flow rate.

The fifth flow rate may be 55 l/min or less, 30 l/min or less, 20 l/min or less, 10 l/min or less, 5 l/min or less or 3 l/min or less. The third fluid flow and/or the fourth fluid flow and/or the fifth fluid flow may be constant fluid flows.

The flow conversion element may be arranged downstream of the fluid conveying element and upstream of the aerosol generator. Further, the flow conversion element may be arranged upstream of the vibrator.

For example, the flow conversion element may be any type of element which is capable of reducing the flow rate of the third fluid flow, so that the fifth flow rate is smaller than the third flow rate. In one embodiment, the flow conversion element may be configured by a bypass. In this case, part of the third fluid flow induced by the fluid conveying element is discharged through the bypass, thus reducing the flow rate of the third fluid flow and thereby converting the third fluid flow into the fifth fluid flow. The part of the third fluid flow discharged by the bypass may be transported outside the device and, for example, discharged into the ambient air.

The bypass may be an orifice, a pipe, a tube, a line or the like. Further, the bypass may comprise one or more valves, such as needle valves, regulators, actuators, interrupters, restrictor elements or the like for controlling the fluid flow through the bypass and thus adjusting the fifth flow rate of the fifth fluid flow. Since, in this embodiment, a single fluid conveying element may be used, the aerosol delivery device has a particularly simple configuration. In particular, the fluid conveying element may be constituted by a pump having an inlet and an outlet, wherein the fourth fluid flow is induced in the device by the inlet of the pump and the third fluid flow is induced in the device by the outlet of the pump. In this case, the fifth flow rate and, thus, also the first flow rate can be adjusted in a simple and reliable manner by suitably choosing or adjusting the flow conversion element, such as a bypass.

In this embodiment, the vibrator may be configured to vibrate the fifth fluid flow but not the fourth fluid flow. In this case, the fourth fluid flow may be identical to the second fluid flow. If the aerosol delivery device is configured in this manner, the flow conversion element can be chosen or adjusted such that the fifth flow rate is smaller than the second flow rate and the pressure fluctuations, i.e., the time variable flow superimposed by the vibrator onto the fifth fluid flow, are compensated by the smaller fifth flow rate of the fifth fluid flow as compared to the second flow rate of the second fluid flow, thus reliably preventing any aerosol deposition in the pharyngeal cavity and the lungs of a patient.

Alternatively, the vibrator may be configured to vibrate the fourth fluid flow so as to induce the second fluid flow in the device and to vibrate the fifth fluid flow so as to induce the first fluid flow in the device.

In this case, the vibrator may be configured so that the vibration of the fourth fluid flow and the vibration of the fifth fluid flow are phase shifted by 180°. In particular, the vibrator may have an outlet which is coupled to the fifth fluid flow and an inlet which is coupled to the fourth fluid flow. For example, a twin head pump as will be described in detail below may be used as the vibrator, wherein the pumping operation of a first piston acts on the fifth fluid flow and the pumping operation of a second piston acts on the fourth fluid flow.

If the fifth flow rate is equal to or smaller than the fourth flow rate and the vibration of the fifth fluid flow and the vibration of the fourth fluid flow are phase shifted, for example, by 180°, it can be ensured in a simple and reliable manner that the first flow rate will be equal to or smaller than the second flow rate throughout an operation cycle or an aerosol deposition cycle of the aerosol delivery device. Hence, an aerosol deposition in the pharyngeal cavity and the lungs of a patient can be reliably prevented, even if the vibration amplitude is significantly larger than the difference between the fifth and fourth flow rates. Therefore, the aerosol delivery device according to this embodiment can be particularly advantageously used for applications in which large vibration amplitudes are required.

The aerosol delivery device of the invention may further comprise at least one restrictor element, such as a nozzle, an orifice, a valve, a restrictor plate, a combination of at least some of these elements or the like, for smoothing the first fluid flow and/or the second fluid flow. In particular, the device may comprise a first restrictor element for smoothing the first fluid flow and a second restrictor element for smoothing the second fluid flow. In this case, the first restrictor element may be arranged upstream of the aerosol generator. Further, the first restrictor element may be arranged upstream of the vibrator.

By smoothing the first fluid flow and/or the second fluid flow, the first and/or second flow rates can be particularly accurately controlled, thus allowing for the prevention of aerosol deposition in the pharyngeal cavity and the lungs of a patient in a particularly reliable and efficient manner.

If the fluid conveying unit comprises a flow conversion element, such as a bypass, the flow conversion element may be arranged upstream of the restrictor element for smoothing the first fluid flow.

The vibrator may be an electromagnetic oscillating unit or a twin head pump, as has been explained above. The twin head pump may comprise first and second pistons which are provided on a drive shaft in such a manner as to be offset by 180°, so that the two pistons operate in opposite directions. Hence, such a pump may be used as a vibrator which is configured so that the vibration of the third fluid flow and the vibration of the fourth fluid flow are phase shifted by 180° or so that the vibration of the fifth fluid flow and the vibration of the fourth fluid flow are phase shifted by 180°, as has been detailed above.

The aerosol delivery device may be configured to stop the first fluid flow before vibrating the aerosol. Further, the aerosol delivery device may be configured to stop the aerosol generation before vibrating the aerosol. This approach allows for a precise control that no aerosol remains inside the device when the vibration is effected. Hence, any aerosol deposition on the inside walls of the device during the aerosol vibrating step can be reliably prevented, thereby further reducing aerosol losses at the walls of the device.

In this way, the unintended deposition of aerosol to locations other than the desired target area, induced by the vibrations, can be significantly reduced. In particular, the impaction of aerosols on the walls of the delivery device can be largely prevented, resulting in a reduced loss of aerosol in the device and consequently an increased aerosol deposition at the desired location, such as the paranasal sinuses.

The invention further provides a method for operating an aerosol delivery device, comprising the steps of generating a predetermined amount of an aerosol in the device, inducing a first fluid flow having a first flow rate in the device for transporting at least a portion of the generated aerosol outside the device, inducing a second fluid flow having a second flow rate in the device for conveying a fluid into the device and vibrating the aerosol, wherein the first flow rate is equal to or smaller than the second flow rate.

The method according to the invention provides the advantageous effects already described in detail above for the device of the invention. In particular, the method allows for an erroneous deposition of aerosols in the pharyngeal cavity and the lungs of a patient to be prevented in a simple and reliable manner.

In one embodiment, a third fluid flow having a third flow rate is induced in the device by a first fluid conveying element, such as a pump, the second fluid flow is induced in the device by a second fluid conveying element, such as a pump, and the third fluid flow is vibrated so as to form or induce the first fluid flow in the device.

The third fluid flow may be vibrated with a predetermined vibration amplitude and the vibration amplitude may be equal to or smaller than the difference between the second and third flow rates.

In another embodiment, a third fluid flow having a third flow rate and a fourth fluid flow having a fourth flow rate are induced in the device by a fluid conveying element, such as a pump, the third fluid flow is vibrated so as to form or induce the first fluid flow in the device and the fourth fluid flow is vibrated so as to form or induce the second fluid flow in the device.

In another embodiment, a third fluid flow having a third flow rate and a fourth fluid flow having a fourth flow rate are induced in the device by a fluid conveying element, such as a pump, the third fluid flow is converted into a fifth fluid flow having a fifth flow rate by a flow conversion element, such as a bypass as described above, and the fifth fluid flow is vibrated so as to form or induce the first fluid flow in the device. The fourth fluid flow may be vibrated so as to induce the second fluid flow in the device.

The method according to the invention may further comprise the step of smoothing the first fluid flow and/or the second fluid flow. In particular, at least one restrictor element, such as a restrictor plate, a nozzle, an orifice, a valve, a combination of at least some of these elements or the like, may be used for smoothing the first fluid flow and/or the second fluid flow.

The method of the invention is a method for operating the aerosol delivery device of the invention. Hence, the further features disclosed in connection with the above description of the device of the invention may also be applied to the method of the invention.

### Brief Description of the Drawings

Hereinafter, non-limiting examples of the invention are explained with reference to the drawings, in which:
- Fig. 1: shows a schematic view of an aerosol delivery device according to a first embodiment of the present invention;
- Fig. 2: shows a schematic view of an aerosol delivery device according to a second embodiment of the present invention;
- Fig. 3: shows a schematic view of an aerosol delivery device according to a third embodiment of the present invention; and
- Fig. 4: shows images of the head of a patient after aerosol deposition using the aerosol delivery device according to the third embodiment of the present invention.

### Detailed Description of Currently Preferred Embodiments

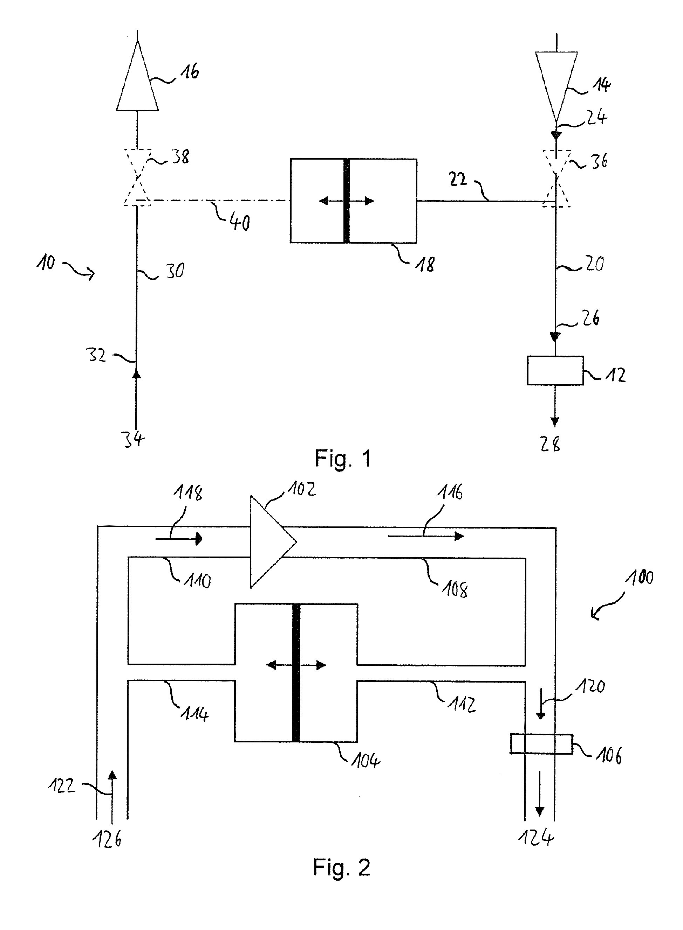

Fig. 1 shows a schematic view of an aerosol delivery device 10 according to a currently preferred first embodiment of the present invention.

The aerosol delivery device 10 shown in Fig. 1 comprises an aerosol generator 12, such as a vibrating membrane nebuliser or the like, a first pump 14 as a first fluid conveying element, a second pump 16 as a second fluid conveying element and a vibrator 18. An outlet of the first pump 14 is connected to the aerosol generator 12 via a pipe 20. An inlet of the first pump 14 may be connected to a gas reservoir or to atmosphere. In the latter case, ambient air is used as the fluid for transporting at least a portion of an aerosol generated in the aerosol generator 12 outside the device 10.

The vibrator 18, such as an electromagnetic oscillating unit or a twin head pump, is connected to the pipe 20 via a pipe 22. The first pump 14 is configured to induce a third fluid flow 24, such as an air flow, having a constant third flow rate, e.g., 10 l/min, in the pipe 20. The vibrator 18 is configured to vibrate the third fluid flow 24, i.e., to superimpose a pressure fluctuation onto the third fluid flow 24, via the pipe 22, thereby forming or inducing a first fluid flow 26 having a first flow rate in the pipe 20. The first fluid flow 26 transports at least a portion of the aerosol generated in the aerosol generator 12 outside the device 10 to the first nostril 28 of a patient, e.g., via a first nosepiece (not shown).

An inlet of the second pump 16 is connected to a pipe 30. An outlet of the second pump 16 may be connected to a gas reservoir or to atmosphere. The second pump 16 is configured to induce a second fluid flow 32 in the pipe 30 for conveying a fluid, i.e., a gas or air, from a second nostril 34 of the patient into the pipe 30, e.g., via a second nosepiece (not shown).

The vibrator 18 is configured to vibrate the third fluid flow 24 with a predetermined vibration amplitude and the vibration amplitude is equal to or smaller than the difference between the second and third flow rates. For example, the third flow rate may be 10 l/min, as has been mentioned above, the second flow rate may be 20 l/min and the vibration amplitude may be 10 l/min or less. In this way, it can be ensured that the first flow rate of the first fluid flow 26 is equal to or smaller than the second flow rate of the second fluid flow 32, so that any erroneous deposition of an aerosol in the pharyngeal cavity and the lungs of the patient can be reliably prevented.

In the aerosol delivery device 10 of the first embodiment shown in Fig. 1, the first pump 14, the second pump 16 and the vibrator 18 form a fluid conveying unit.

As is indicated by dashed lines in Fig. 1, the aerosol delivery device 10 of the first embodiment of the present invention may further comprise a first 36 and/or a second restrictor element 38 arranged in the pipe 20 and/or the pipe 30, respectively, for smoothing the first fluid flow 26 and/or the second fluid flow 32, respectively. The first restrictor element 36 and/or the second restrictor element 38 may be a nozzle, an orifice, a restrictor plate or the like.

Moreover, as is indicated by a dash-dot line in Fig. 1, the vibrator 18 may further be connected to the pipe 30 through a pipe 40. In this case, the vibrator 18 is configured to vibrate both the third fluid flow 24 in the pipe 20 and, simultaneously, a fluid flow in the pipe 30. Specifically, in this case, the second pump 16 is configured to induce a fourth fluid flow having a fourth flow rate in the pipe 30 and the vibrator 18 is configured to vibrate the fourth fluid flow so as to form or induce the second fluid flow 32 in the device 10. The vibrations imparted by the vibrator 18 to the third fluid flow 24 and the fourth fluid flow are phase shifted by 180°.

Hence, by choosing a third flow rate which is equal to or smaller than the fourth flow rate, it can be ensured that the first flow rate is equal to or smaller than the second flow rate throughout an operation cycle of the aerosol delivery device 10, thereby enabling a reliable prevention of erroneous aerosol depositions in the pharyngeal cavity and the lungs of the patient. In such a device configuration, a twin head pump may be particularly advantageously used as the vibrator 18.

As has been explained above, the third flow rate of the third fluid flow 24 may be smaller than the fourth flow rate of the fourth fluid flow. This can be achieved, for example, by using a first pump 14 which has a smaller pumping capacity than the second pump 16.

Figure 2 shows a schematic view of an aerosol delivery device 100 according to a currently preferred second embodiment of the present invention.

The aerosol delivery device 100 shown in Fig. 2 comprises a pump 102 as a fluid conveying element, a vibrator 104, such as an electromagnetic oscillating unit or a twin head pump, and an aerosol generator 106, such as a vibrating membrane nebuliser or the like. An outlet of the pump 102 is connected to the aerosol generator 106 through a pipe 108 and an inlet of the pump 102 is connected to a pipe 110. The vibrator 104 is connected to the pipe 108 through a pipe 112 and to the pipe 110 through a pipe 114.

The pump 102 is configured to induce a third fluid flow 116 having a third flow rate in the pipe 108 and a fourth fluid flow 118 having a fourth flow rate in the pipe 110. Since the single pump 102 is used as the fluid conveying element, the third flow rate is equal to the fourth flow rate. Further, the third and fourth flow rates are constant.

The vibrator 104 is configured to vibrate the third fluid flow 116 so as to form or induce a first fluid flow 120 having a first flow rate in the pipe 108 and to vibrate the fourth fluid flow 118 so as to form or induce a second fluid flow 122 in the pipe 110. The first fluid flow 120, such as a gas flow or an air flow, transports at least a portion of the aerosol generated in the aerosol generator 106 outside the device 100 to a first nostril 124 of a patient. The second fluid flow 122 conveys a fluid from a second nostril 126 of the patient into the device 100. Hence, the aerosol delivery device 100 forms a closed fluid circuit with the patient.

The vibrations imparted to the third fluid flow 116 and the fourth fluid flow 118 by the vibrator 104 are phase shifted by 180°. Hence, it can be ensured that throughout an operation cycle of the device 100, the first flow rate is equal to the second flow rate. Therefore, an erroneous deposition of aerosols in the pharyngeal cavity and the lungs of the patient can be reliably prevented.

In the aerosol delivery device 100 according to the second embodiment of the present invention shown in Fig. 2, the pump 102 and the vibrator 104 form a fluid conveying unit.

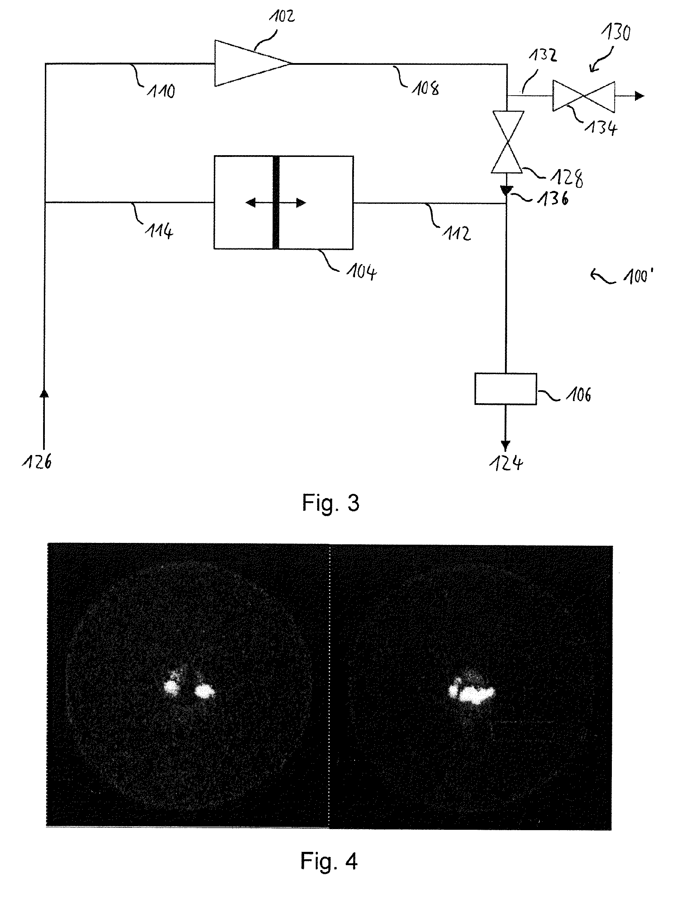

Figure 3 shows a schematic view of an aerosol delivery device 100' according to a currently preferred third embodiment of the present invention. The configuration of the aerosol delivery device 100' according to the third embodiment is similar to that of the aerosol delivery device 100 according to the second embodiment of the present invention. Hence, the same reference signs have been used to designate the same or similar elements thereof.

The aerosol delivery device 100' shown in Fig. 3 comprises a pump 102 as a fluid conveying element, a vibrator 104, such as an electromagnetic oscillating unit or a twin head pump, and an aerosol generator 106, such as a vibrating membrane nebuliser or the like. Substantially, the aerosol delivery device 100' has the same components and operates in the same manner as the aerosol delivery device 100 shown in Fig. 2.

However, the aerosol delivery device 100' differs from the aerosol delivery device 100 in that it further comprises a restrictor element 128 arranged in the pipe 108, such as a nozzle, an orifice, a restrictor plate, a valve or the like, for smoothing the first fluid flow 120 and a bypass 130. The bypass 130 forms a flow conversion element. The bypass 130 can be formed by a pipe 132 connected to the pipe 108 and a restrictor element 134, such as a nozzle, an orifice, a restrictor plate, a valve or the like, arranged in the pipe 132. The bypass including restrictor can also be realised by a defined or adjustable opening in the pipe 108 upstream of the restrictor 128.

The pump 102 is configured to induce a third fluid flow 116 having a third flow rate in the pipe 108 and a fourth fluid flow 118 having a fourth flow rate in the pipe 110 (see Fig. 2), as has been explained in detail above. A portion of the third fluid flow 116 flows through the bypass 130 outside the device 100', e.g., into atmosphere, thereby converting the third fluid flow 116 into a fifth fluid flow 136 having a fifth flow rate which is smaller than the third flow rate of the third fluid flow 116. The vibrator 104 is configured to vibrate the fifth fluid flow 136 so as to form or induce the first fluid flow 120 in the pipe 108.

The vibrations imparted by the vibrator 104 to the fifth fluid flow 136 and the fourth fluid flow 118 are phase shifted by 180°. Since the fifth flow rate of the fifth fluid flow 136 is smaller than the third flow rate of the third fluid flow 116 and, thus, also smaller than the fourth flow rate of the fourth fluid flow 118, it is ensured that the first flow rate of the first fluid flow 120 is smaller than the second flow rate of the second fluid flow 122 throughout an operation cycle of the aerosol delivery device 100'. In this way, an erroneous aerosol deposition in the pharyngeal cavity and the lungs of a patient can be prevented in a particularly reliable and efficient manner.

In the aerosol delivery device 100' according to the third embodiment of the present invention, the pump 102, the vibrator 104 and the bypass 130 form a fluid conveying unit.

The aerosol delivery device 100' according to the third embodiment of the invention shown in Fig. 3 was used to carry out experimental trials which will be described in detail in the following.

A twin head pump of the type BOXER 5102 supplied by BOXERPUMPS was used as the vibrator 104 with a vibration amplitude of approximately 10 l/min and a frequency of 66 Hz. The inlet and outlet valves of this pump were removed. A pipe 132 with a needle valve (not shown) was used as the bypass 130. By using such a bypass 130, the fifth flow rate of the fifth fluid flow 136 was reduced to 1.8 l/min, while the fourth flow rate of the fourth fluid flow 118 was 3.0 l/min. Air was used as the fluid.

The experimental trials were conducted using the PARI Vibrent aerosol generation device in a combined aerosol delivery mode as explained in detail in EP-A-2 380 618, i.e., in an aerosol delivery mode in which the generated aerosol is transported with an intermittent transport flow and subsequently the transported aerosol is vibrated after the aerosol generation and the aerosol transport have been stopped.

The pipe 108 was tightly connected to the first nostril 124 of a proband via a first nosepiece and the pipe 110 was tightly connected to the second nostril 126 of the proband via a nose filter, a nose resistor and a second nosepiece, thus forming a closed air circuit. During operation of the device 100', the proband breathed through his mouth via a mouth filter, so as to allow for the exhaled amount of aerosol to be determined. One aerosol delivery cycle with a duration of approximately 1 min was performed for each nostril 124, 126 of the proband. A radioactively marked aerosol was used in order to enable measurement of the amount of aerosol deposited in the nose and the lungs.

After completion of the aerosol delivery cycles, the aerosol amount in the nose filter, the mouth filter, the proband's head and the proband's abdomen were measured so as to determine the fractions of aerosol deposited in the proband's nose and lungs as well as the amount of aerosol exhaled by the proband and the amount of aerosol conveyed back into the aerosol delivery device 100' by the second fluid flow 122.

For determining the aerosol fraction deposited in the paranasal sinuses, the proband's nose was shielded by means of a lead mask and frontal and lateral images of the proband's head were taken. Examples of such frontal images are shown in Fig. 4, wherein the image on the right-hand side in Fig. 4 was taken without any shielding and the image on the left-hand side in Fig. 4 was taken with a lead mask used for shielding the proband's nose.

The experimental trials described above were carried out with three different probands. The results of these trials are shown in Table 1, wherein the fractions of the nasal deposition and the lung deposition, the exhaled amount of aerosol and the amount of aerosol conveyed back into the device 100' by the second fluid flow 122 are given as percentages of the total aerosol output of the aerosol delivery device 100' and the fraction of the aerosol deposition in the paranasal sinuses is given as a percentage of the total nasal deposition.

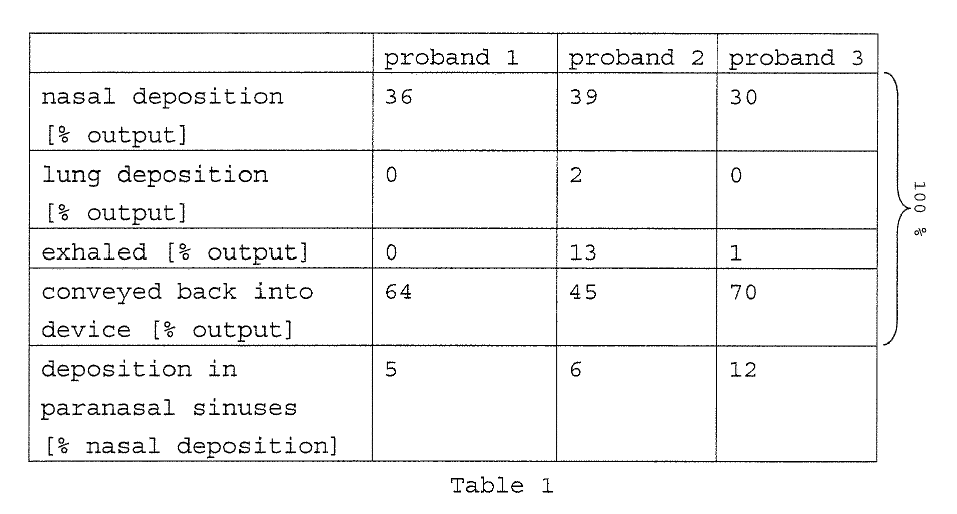

The percentage values for the fractions of the nasal deposition and the lung deposition, the exhaled amount of aerosol and the amount of aerosol conveyed back into the device 100' by the second fluid flow 122 add up to a total of 100%. Any deviation from this total in Table 1 is due to rounding errors.

As can be seen from the results shown in Table 1, a sizeable aerosol deposition in the nose, in particular in the paranasal sinuses, could be achieved, while no significant aerosol deposition in the lungs was observed. Specifically, the aerosol deposition in the lungs was found to be zero for both the first and the third proband and 2% of the total aerosol output for the second proband. However, this result for the second proband is believed to be caused by a programming error.

The measurement values of the exhaled amounts of aerosol obtained for the first and third probands further indicate the absence of any significant aerosol deposition in the pharyngeal cavity.

The experimental results presented above demonstrate that an undesired deposition of aerosols in the pharyngeal cavity and the lungs of a patient can be prevented by the aerosol delivery device and the method of the present invention without the requirement of a closed soft palate, even if the patient breathes during the aerosol delivery.

## Claims

1. An aerosol delivery device (10, 100, 100') comprising:
- an aerosol generator (12, 106) for generating an aerosol in the device, and
- a fluid conveying unit (14, 16, 18; 102, 104, 130) configured to induce a first fluid flow (26, 120) having a first flow rate in the device for transporting at least a portion of the generated aerosol outside the device and to induce a second fluid flow (32, 122) having a second flow rate in the device for conveying a fluid into the device,
wherein the fluid conveying unit comprises a vibrator (18, 104) for vibrating the aerosol, and
the first flow rate is equal to or smaller than the second flow rate.

2. The aerosol delivery device (10) according to claim 1, wherein the fluid conveying unit further comprises a first fluid conveying element (14) configured to induce a third fluid flow (24) having a third flow rate in the device and a second fluid conveying element (16) configured to induce the second fluid flow (32) in the device, and the vibrator (18) is configured to vibrate the third fluid flow (24) so as to induce the first fluid flow (26) in the device.

3. The aerosol delivery device (10) according to claim 2, wherein the vibrator (18) is configured to vibrate the third fluid flow (24) with a predetermined vibration amplitude and the vibration amplitude is equal to or smaller than the difference between the second and third flow rates.

4. The aerosol delivery device (100) according to claim 1, wherein the fluid conveying unit further comprises a fluid conveying element (102) configured to induce a third fluid flow (116) having a third flow rate and a fourth fluid flow (118) having a fourth flow rate in the device, and the vibrator (104) is configured to vibrate the third fluid flow (116) so as to induce the first fluid flow (120) in the device and to vibrate the fourth fluid flow (118) so as to induce the second fluid flow (122) in the device.

5. The aerosol delivery device (100') according to claim 1, wherein the fluid conveying unit further comprises a fluid conveying element (102) configured to induce a third fluid flow (116) having a third flow rate and a fourth fluid flow (118) having a fourth flow rate in the device and a flow conversion element (130) configured to convert the third fluid flow (116) into a fifth fluid flow (136) having a fifth flow rate and wherein the vibrator (104) is configured to vibrate the fifth fluid flow (136) so as to induce the first fluid flow (120) in the device.

6. The aerosol delivery device (100') according to claim 5, wherein the vibrator (104) is configured to vibrate the fourth fluid flow (118) so as to induce the second fluid flow (122) in the device.

7. The aerosol delivery device (10, 100, 100') according to any one of the preceding claims, further comprising at least one restrictor element (36, 38) for smoothing the first fluid flow (26, 120) and/or the second fluid flow (32, 122).

8. The aerosol delivery device (10, 100, 100') according to any one of the preceding claims, wherein the vibrator (18, 104) is an electromagnetic oscillating unit or a twin head pump.

9. A method for operating an aerosol delivery device (10, 100, 100'), comprising the steps of:
- generating a predetermined amount of an aerosol in the device,
- inducing a first fluid flow (26, 120) having a first flow rate in the device for transporting at least a portion of the generated aerosol outside the device,
- inducing a second fluid flow (32, 122) having a second flow rate in the device for conveying a fluid into the device, and
- vibrating the aerosol,
wherein the first flow rate is equal to or smaller than the second flow rate.

10. The method according to claim 9, wherein a third fluid flow (24) having a third flow rate is induced in the device by a first fluid conveying element (14), the second fluid flow (32) is induced in the device by a second fluid conveying element (16) and the third fluid flow (24) is vibrated so as to induce the first fluid flow (26) in the device.

11. The method according to claim 10, wherein the third fluid flow (24) is vibrated with a predetermined vibration amplitude and the vibration amplitude is equal to or smaller than the difference between the second and third flow rates.

12. The method according to claim 9, wherein a third fluid flow (116) having a third flow rate and a fourth fluid flow (118) having a fourth flow rate are induced in the device by a fluid conveying element (102), the third fluid flow (116) is vibrated so as to induce the first fluid flow (120) in the device and the fourth fluid flow (118) is vibrated so as to induce the second fluid flow (122) in the device.

13. The method according to claim 9, wherein a third fluid flow (116) having a third flow rate and a fourth fluid flow (118) having a fourth flow rate are induced in the device by a fluid conveying element (102), the third fluid flow (116) is converted into a fifth fluid flow (136) having a fifth flow rate by a flow conversion element (130) and the fifth fluid flow (136) is vibrated so as to induce the first fluid flow (120) in the device.

14. The method according to claim 13, wherein the fourth fluid flow (118) is vibrated so as to induce the second fluid flow (122) in the device.

15. The method according to any one of claims 9 to 14, further comprising the step of smoothing the first fluid flow (26, 120) and/or the second fluid flow (32, 122).
